## Europäisches Patentamt

## European Patent Office

## Office européen des brevets

(11) Veröffentlichungsnummer: **0 105 098**
**B1**

(12) # EUROPÄISCHE PATENTSCHRIFT

(45) Veröffentlichungstag der Patentschrift:
04.06.86

(51) Int. Cl.⁴: **A 61 F 2/32**

(21) Anmeldenummer: **83106646.9**

(22) Anmeldetag: **07.07.83**

(54) **Künstliche Hüftgelenkpfanne.**

(30) Priorität: **05.10.82 CH 5839/82**

(43) Veröffentlichungstag der Anmeldung:
**11.04.84 Patentblatt 84/15**

(45) Bekanntmachung des Hinweises auf die Patenterteilung:
**04.06.86 Patentblatt 86/23**

(84) Benannte Vertragsstaaten:
**AT BE DE FR GB IT NL**

(56) Entgegenhaltungen:
**DE - A - 2 537 644**
**DE - A - 2 823 306**
**DE - A - 2 836 835**
**FR - A - 2 183 233**
**GB - A - 1 430 071**
**US - A - 3 683 421**

(73) Patentinhaber: **GEBRÜDER SULZER AKTIENGESELLSCHAFT, Zürcherstrasse 9, CH-8401 Winterthur (CH)**

(72) Erfinder: **Weber, Bernard, Prof. Dr. med., Kantonsspital, CH-9007 St. Gallen (CH)**
Erfinder: **Frey, Otto, Walrütistrasse 56, CH-8400 Winterthur (CH)**

(74) Vertreter: **Dipl.-Ing. H. Marsch Dipl.-Ing. K. Sparing Dipl.-Phys.Dr. W.H. Röhl Patentanwälte, Rethelstrasse 123, D-4000 Düsseldorf (DE)**

ACTORUM AG

## Beschreibung

Die Erfindung betrifft eine künstliche Hüftgelenkpfanne zur Verankerung im Beckenknochen mit Hilfe eines aushärtenden Knochenzements, die mit über den Umfang ihres Randbereichs verteilen Bohrungen versehen ist, die im wesentlichen parallel zu ihrer Achse verlaufen.

Künstliche Hüftgelenkpfannen, die in ihrem Randbereich verteilte Bohrungen aufweisen, sind bekannt (FR-A-2 183 233; US-A-3 943 576). Diese Hüftgelenkpfannen sind für eine knochenzementfreie Verankerung im Becken bestimmt; die in ihrem Rand verteilten Bohrungen haben die Aufgabe, das Einwachsen von Gewebe zu ermöglichen.

Demgegenüber ist die Hüftgelenkpfanne gemäss der Erfindung für eine Verankerung bestimmt, bei der sie im wesentlichen durch einen aushärtenden Knochenzement gehalten wird. Beim Einsetzen derartiger Pfannen ergeben sich häufig Schwierigkeiten dadurch, dass der zähflüssige Knochenzement, der im allgemeinen im Überschuss in die operativ geschaffene Ausnehmung des Beckenknochens eingefüllt wird, nicht entweichen kann. Er fliesst dann über den seitlichen Rand der eingepressten Pfanne; dabei gelangt er häufig in den Gelenkbereich der Prothese, wo er zu Beschädigungen der Gleitflächen des künstlichen Gelenks Anlass geben kann.

Aufgabe der Erfindung ist es, eine Hüftgelenkpfanne zu schaffen, bei der diese Schwierigkeiten überwunden werden, und die Arbeit des Operateurs erleichtert wird, indem der überschüssige Knochenzement kontrolliert und geführt aus der Ausnehmung des Beckens abfliessen kann. Die Lösung dieser Aufgabe erfolgt dadurch, dass sich die Bohrungen mindestens auf einem Teil ihres Verlaufs zur freien Oberfläche der Gelenkpfanne hin konisch verengen.

Die konische Form der Bohrungen hat eine düsenähnliche Wirkung auf den «Strom» des zähflüssigen Knochenzements und eröffnet dem überschüssigen Zement «gepfadete» Wege. Dieser tritt daher in einer Vielzahl von wurmartigen Stromfäden aus dem Rand der Gelenkpfanne aus und kann von diesem glatten, ebenen und relativ festen Rand leicht entfernt werden.

Schliesst sich an die Verengung der Bohrungen zur freien Oberfläche der Pfanne hin ein sich nach aussen erweiternder Gegenkonus an, so wirken — nach Aushärten des Knochenzements — die damit gefüllten Bohrungen als Nietverbindungen und erhöhen die feste Verbindung zwischen Zement und Pfanne.

Im folgenden wird die Erfindung anhand eines Ausführungsbeispiels im Zusammenhang mit der Zeichnung näher erläutert.

Fig. 1 zeigt eine Ausführungsform der neuen Hüftgelenkpfanne in einem Schnitt I-I von Fig. 2, der in einer Meridianebene verläuft;

Fig. 2 ist eine Aufsicht auf Fig. 1 von oben;

Fig. 3 schliesslich zeigt in vergrössertem Massstab eine der erfindungsgemäss ausgebildeten Bohrungen in einer, mittels Knochenzement in einem Knochen verankerten Gelenkpfanne.

Die künstliche Hüftgelenkpfanne 1, die beispielsweise aus Kunststoff, insbesondere Polyäthylen, besteht, ist in ihrer Aussenform im wesentlichen eine Halbkugel, die äquatorseitig durch einen Rand 2 abgeschlossen ist. In den Pfannenkörper ist eine ebenfalls halbkugelförmige Schale 3 eingearbeitet, in die der nicht gezeigte Kugelkopf der Femurprothese eingreift. Oberhalb des Randes 2 ist eine Rille 4 in den Pfannenkörper eingestochen, in die als Röntgenkontrast-Element ein Metalldrahtring 5 eingelegt ist.

Über den Umfang des Randes 2 sind zur Achse der Gelenkpfanne 1 parallele Bohrungen 6 verteilt, die sich erfindungsgemäss in ihrem Verlauf zur freien Oberfläche der Gelenkpfanne 1 hin zunächst konisch verengen. Die Verengung endet etwa in der Mittelebene 7 der Randdicke; von dieser aus erweitern sich die Bohrungen in Richtung auf die freie Oberfläche der Pfanne 1 wieder in einem Gegenkonus 8.

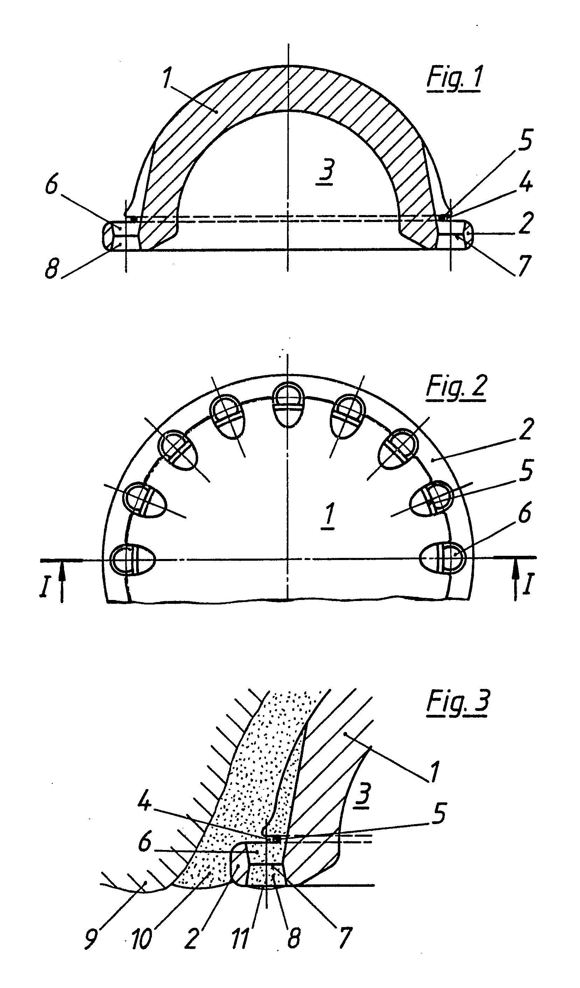

Wie Fig. 3 zeigt, sind die Bohrungen 6 bei einer in ein Becken 9 eingesetzten Pfanne 1 mit Knochenzement 10 gefüllt, der einen etwa von der Mitte der Randdicke zur freien Oberfläche der Pfanne 1 verlaufenden, nietenartigen Pfropfen 11 bildet.

## Patentansprüche

1. Künstliche Hüftgelenkpfanne zur Verankerung im Beckenknochen mit Hilfe eines aushärtenden Knochenzements, die mit über den Umfang ihres Randbereichs verteilten Bohrungen versehen ist, die im wesentlichen parallel zu ihrer Achse verlaufen, dadurch gekennzeichnet, dass sich die Bohrungen (6) mindestens auf einem Teil ihres Verlaufs zur freien Oberfläche der Gelenkpfanne (1) hin konisch verengen.

2. Hüftgelenkpfanne nach Anspruch 1, dadurch gekennzeichnet, dass an die Verengung zur freien Oberfläche der Gelenkpfanne (1) hin ein sich erweiternder Gegenkonus (8) anschliesst.

## Claims

1. A prosthetic acetabulum for anchorage in the pelvis by means of a curable bone cement, the acetabulum being formed with bores distributed over the periphery of its edge zone and extending substantially parallel to its axis, characterised in that the bores (6) narrow conically at least in a part of their extent towards the free surface of the acetabulum (1).

2. An acetabulum according to claim 1, characterised in that the narrowing towards the free surface of the acetabulum (1) merges into a widening and oppositely directed cone (87.

## Revendications

1. Cavité coxo-fémorale artificielle destinée à être ancrée dans l'os du bassin à l'aide d'un ciment osseux qui durcit et qui est munie de perforations distribuées sur la périphérie de sa zone marginale et qui s'étendent sensiblement parallèlement à l'axe, caractérisée en ce que les perforations (6) se rétrécissent coniquement au moins sur une partie de leur dimension vers la surface libre de la cavité d'articulation (1).

2. Cavité coxo-fémorale artificielle selon la revendication 1, caractérisée en ce qu'une contre-cône (8) qui s'élargit se raccorde sur le rétrécissement allant vers la surface libre de la cavité d'articulation (17.

Fig. 1

Fig. 2

Fig. 3